# EUROPEAN PATENT APPLICATION

(11) **EP 4 785 970 A1**
(43) Date of publication of application: **05.08.2026**
(21) Application number: 25154642.0
(22) Date of filing: 29.01.2025
(51) Int. Cl.: A61M 1/00

(54) **NEGATIVE PRESSURE WOUND THERAPY**

(71) Applicant: Mölnlycke Health Care AB, 431 21 Mölndal (SE)
(72) Inventor: BENGTSSON, Erik, 431 41 Mölndal (SE); UVEBORN, Johan, 436 40 Askim (SE); CARLSSON, Matthias, 431 68 Mölndal (SE)
(74) Representative: Zacco Sweden AB

(57) **Abstract**

An apparatus and related aspects for providing reduced pressure to a wound site are disclosed. The apparatus comprises a source of negative pressure configured to provide negative pressure via a first fluid flow path from an inlet of the source of negative pressure to the wound site covered by a wound dressing. The apparatus further comprises a pressure sensor configured to monitor a pressure level in a fluid flow path that is in fluid connection with the wound site or to monitor a pressure level at the wound site, and control circuitry operatively connected to the source of negative pressure and to the pressure sensor. The control circuitry is configured to activate the source of negative pressure in order to establish a set target pressure at the wound site, and store a plurality of pressure values, as measured by the pressure sensor, while the source of negative pressure is active. The control circuitry is further configured to configure one or more control parameters of the apparatus based at least in part on the stored plurality of pressure values.

## Description

### TECHNICAL FIELD

The herein disclosed embodiments generally relate to negative pressure wound therapy (NPWT). In particular, the herein disclosed embodiments relate to apparatuses for providing reduced pressure to a wound site, methods for controlling an apparatus for providing reduced pressure to a wound site, and thereto related computer program products and computer-readable storage media.

### BACKGROUND

Negative pressure wound therapy (NPWT) is a technique that promotes healing of e.g. surgical, acute and chronic wounds by the application of a sub-atmospheric pressure ("negative pressure") to the wound site, using a negative pressure pump. Wound healing is achieved by applying a negative pressure, such as "vacuum" through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site.

The effectiveness of NPWT may depend on the compatibility between the NPWT device and the wound dressing used. However, variations in wound types, anatomical locations, and exudate levels necessitate the use of different dressing materials and configurations. Many current NPWT devices may be limited in their versatility and requiring users to rely on proprietary systems or incompatible components. This lack of flexibility can hinder effective wound management, increase costs, and reduce patient access to care.

To address these challenges, there is a need for innovative NPWT devices that can seamlessly integrate with a wide range of scenarios while maintaining performance, durability, and ease of use.

### SUMMARY

The herein disclosed technology seeks to mitigate, alleviate or eliminate one or more of the above-identified deficiencies and disadvantages in the prior art to address various problems relating the configuration of NPWT devices.

Various aspects and embodiments of the disclosed technology are defined below and in the accompanying independent and dependent claims.

An aspect of the disclosed technology comprises an apparatus for providing reduced pressure to a wound site. The apparatus comprises a source of negative pressure configured to provide negative pressure via a first fluid flow path from an inlet of the source of negative pressure to the wound site covered by a wound dressing. The apparatus further comprises a pressure sensor configured to monitor a pressure level in a fluid flow path that is in fluid connection with the wound site or to monitor a pressure level at the wound site, and control circuitry operatively connected to the source of negative pressure and to the pressure sensor. The control circuitry is configured to activate the source of negative pressure in order to establish a set target pressure at the wound site, and store a plurality of pressure values, as measured by the pressure sensor, while the source of negative pressure is active. The control circuitry is further configured to configure one or more control parameters of the apparatus based at least in part on the stored plurality of pressure values.

Another aspect of the disclosed technology comprises a computer-implemented method for controlling an apparatus for providing reduced pressure to a wound site according to any one of the embodiments disclosed herein. The computer-implemented method comprises activating the source of negative pressure in order to establish a set target pressure at the wound site, and storing a plurality of pressure values, as measured by the pressure sensor, while the source of negative pressure is active. The computer-implemented method further comprises configuring one or more control parameters of the apparatus based at least in part on the stored plurality of pressure values. With this aspect of the disclosed technology, similar advantages and preferred features are present as in the other aspects.

Another aspect of the disclosed technology comprises a computer program product comprising instructions which, when the program is executed by a computing device of an apparatus for providing reduced pressure to a wound site, causes the apparatus to carry out the method according to any one of the embodiments disclosed herein. With this aspect of the disclosed technology, similar advantages and preferred features are present as in the other aspects.

Another aspect of the disclosed technology comprises a (non-transitory) computer-readable storage medium comprising instructions which, when executed by a computing device of an apparatus for providing reduced pressure to a wound site, causes the apparatus to carry out the method according to any one of the embodiments disclosed herein. With this aspect of the disclosed technology, similar advantages and preferred features are present as in the other aspects.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

Another aspect of the disclosed technology comprises a comprises a wound treatment system comprising apparatus according to any one of the embodiments disclosed herein, a wound cover for creating a sealed space defined in part by the wound site, and a tubing assembly defining the fluid flow path. With this aspect of the disclosed technology, similar advantages and preferred features are present as in the other aspects.

The disclosed aspects and preferred embodiments may be suitably combined with each other in any manner apparent to anyone of ordinary skill in the art, such that one or more features or embodiments disclosed in relation to one aspect may also be considered to be disclosed in relation to another aspect or embodiment of another aspect.

As mentioned, NPWT devices have become widely utilized in wound care due to their ability to promote healing through the application of controlled negative pressure to a wound site. These devices are typically used in conjunction with various types of wound dressings, each designed to address specific wound conditions such as size, shape, exudate levels, and location. However, the adaptability of a single NPWT device to effectively operate with a range of different wound dressings remains a significant challenge.

Current NPWT devices are often designed with a fixed or limited configuration, making it difficult or impossible to optimize their functionality across all types of wound dressings. This mismatch can result in suboptimal negative pressure distribution, reduced efficiency in fluid removal, and potentially compromised wound healing outcomes. Additionally, the lack of flexibility in device configuration may require caregivers to rely on multiple devices or components, increasing the complexity and cost of wound management.

To this end, embodiments disclosed herein present an NPWT device that is capable of automatically configuring or adapting itself to work effectively with a variety of wound dressings, and potentially providing improved performance across a wider range of wound characteristics or dressings.

An advantage of some embodiments is that the handling of NPWT devices may be facilitated thereby improving the user experience.

An advantage of some embodiments is that the NPWT device is automatically configurable for different types of wound dressings thereby providing a more optimized therapy for a variety of different types of wound dressings.

An advantage of some embodiments is that the NPWT device consumes less energy by reducing the frequency of unnecessary activations of the source of negative pressure due to having a more optimized configuration for the applied wound dressing, thereby requiring battery replacements less often and reducing the carbon footprint of the NPWT device.

An advantage of some embodiments is that the NPWT devices is enabled to correct an inadvertently set erroneous configuration and thereby improve patient safety.

Further embodiments are defined in the dependent claims. It should be emphasized that the term "comprises/comprising" when used in this specification is taken to specify the presence of stated features, integers, steps, or components. It does not preclude the presence or addition of one or more other features, integers, steps, components, or groups thereof.

These and other features and advantages of the disclosed technology will in the following be further clarified with reference to the embodiments described hereinafter.

### BRIEF DESCRIPTION OF THE DRAWINGS

The above aspects, features and advantages of the disclosed technology, will be more fully appreciated by reference to the following illustrative and non-limiting detailed description of example embodiments of the present disclosure, when taken in conjunction with the accompanying drawings, in which:
Fig. 1 is a schematic illustration of a wound treatment system in accordance with some embodiments.
Fig. 2 is a schematic illustration of a wound treatment system in accordance with some embodiments.
Fig. 3A is a schematic illustration of a plot of a plurality of pressure values as measured by a pressure sensor of an apparatus for providing reduced pressure to a wound site in accordance with some embodiments.
Fig. 3B is a schematic illustration of a pressure curve profile fitted to the plot of the plurality of pressure values from Fig. 3A in accordance with some embodiments.
Fig. 4A is a schematic illustration of a set of pressure curve profiles for a set of predefined types of wound dressings in accordance with some embodiments.
Fig. 4B is a schematic illustration of a comparison between the pressure curve profile from Fig. 3B and the set of pressure curve profiles for the set of predefined types of wound dressings from Fig. 4A in accordance with some embodiments.
Fig. 5 is a schematic flowchart representation of a method for operating an apparatus for providing negative pressure to a wound site in accordance with some embodiments.

### DETAILED DESCRIPTION

The present disclosure will now be described in detail with reference to the accompanying drawings, in which some example embodiments of the disclosed technology are shown. The disclosed technology may, however, be embodied in other forms and should not be construed as limited to the disclosed example embodiments. The disclosed example embodiments are provided to fully convey the scope of the disclosed technology to the skilled person. Like reference characters refer to like elements throughout. Those skilled in the art will appreciate that the steps, services and functions explained herein may be implemented using individual hardware circuitry, using software functioning in conjunction with hardware circuitry such as a programmed microprocessor or general-purpose computer, using one or more Application Specific Integrated Circuits (ASICs), using one or more Field Programmable Gate Arrays (FPGA) and/or using one or more Digital Signal Processors (DSPs).

It will also be appreciated that when the present disclosure is described in terms of a method, it may also be embodied in an apparatus comprising one or more processors, one or more memories coupled to the one or more processors, where computer code is loaded to implement the method. For example, the one or more memories may store one or more computer programs that cause the apparatus to perform the steps, services and functions disclosed herein when executed by the one or more processors in some embodiments.

It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. Similarly, "at least one of A and B" is to be interpreted as only A, only B, or both A and B.

It will also be understood that, although the term first, second, etc. may be used herein to describe various elements or features, these elements should not be limited by these terms. These terms are only used to distinguish one element from another. For example, a first signal could be termed a second signal, and, similarly, a second signal could be termed a first signal, without departing from the scope of the embodiments. The term "operatively connected" is in the context of the present disclosure to be understood as that the "operatively connected" entities or units can transmit and/or receive signals to and/or from each other.

As used herein, the term "in response to" may be construed to mean "when or "upon" or "if" depending on the context. Similarly, the phrase "if it is determined' or "when it is determined" or "in an instance of" may be construed to mean "upon determining or "in response to determining" or "upon detecting and identifying occurrence of an event" or "in response to detecting occurrence of an event" depending on the context. Accordingly, the phrase "if X equals Y" may be construed as "when X equals Y", "when it is determined that X equals Y", "in response to X being equal to Y", or "in response to detecting/determining that X equals Y" depending on the context.

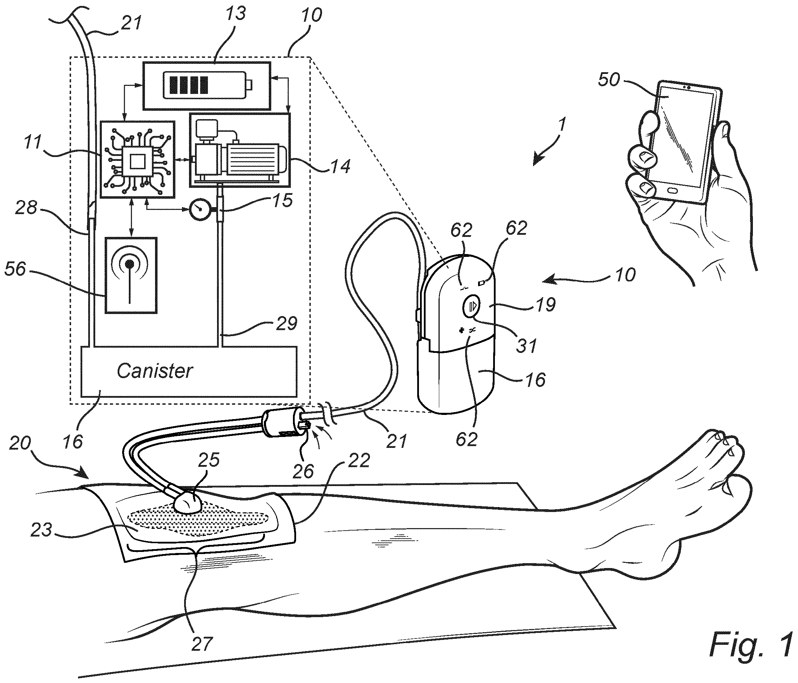

Turning now to the drawings, and to Fig. 1 in particular, there is schematically illustrated a wound treatment system 1 (sometimes simply referred to as "the system" 1) in accordance with some embodiments. The depicted wound treatment system 1 may be referred to as an "NPWT system" 1, "reduced pressure wound treatment system" 1, or "negative pressure wound treatment system" 1. The wound treatment system 1 comprises an apparatus 10 for providing reduced pressure to a tissue site 27 (or otherwise referred to as a wound site 27).

The wound treatment system 1 further comprises a wound cover 22 that is adapted to create a sealed space 23 defined in part by a wound surface, such as at the skin of a user/person, at or around a wound of the user/person. Further, the apparatus 10 (may also be referred to as an NPWT device 10) is fluidly connected to the wound cover 22 using e.g. a tubing 21. The tubing 21 may be of any suitable flexible tubing fabricated from elastomeric or polymeric materials. The tubing 21 may connect to the wound dressing 20, or more specifically to the wound cover 22 of the wound dressing 20, via a suitable connector 25. The connector 25 may be adhered or otherwise attached to the wound cover 22. In particular, the connector 25 may be attached around an opening (not shown) formed in the wound cover 22. The tubing 21 may comprise one, two, or more individual tubes.

The expression "wound cover" as used herein should be interpreted broadly as any wound site member, e.g. it can be a film sealed around a periphery of a wound site 27, where a wound filler may be used to fill the wound volume prior to the application of such wound cover. Wound cover may also refer to a backing layer of a wound dressing 20 comprising an additional layer(s) such as for example an absorbent layer and/or a spacer layer. The wound cover 22 may be a moisture vapour permeable layer, and have a a moisture vapor transmission rate (MVTR). The MVTR is the rate at which the backing layer (and thus also the dressing) allows moisture to evaporate. The MVTR is measured by the standard method NWSP070.4R0(15). The MVTR is measured at a temperature of 38°C.

The absorbent layer of the wound dressing 20 may be in the form of an absorbent structure (not shown) comprising one or more layers where at least one of these layers is a superabsorbent layer comprising superabsorbent polymer (SAP) particles. A "superabsorbent polymer" or "SAP" is a polymer that can absorb up to 300 times its own weight in aqueous fluids. Superabsorbent polymers are constituted by water-swellable and water insoluble polymers capable of absorbing large quantities of fluid upon formation of a hydrogel. The superabsorbent polymers for use in accordance with the present disclosure may be inorganic or organic crosslinked hydrophilic polymers, such as polyvinyl alcohols, polyethylene oxides, crosslinked polyacrylates and the like. Typically, the SAP particles comprise sodium acrylate. The SAP material is in the form of particles. The size of the superabsorbent particles may be in the range of from 45 to 850 µm, e.g. from 150 to 600 µm.

The apparatus 10 comprises a source of negative pressure 14 configured to provide negative pressure (i.e. sub-atmospheric pressure) via a fluid flow path from an inlet of the source of negative pressure 14 to the wound dressing 20, or more specifically to provide negative pressure under a wound cover. In some embodiments, the source of negative pressure 14 comprises a negative pressure pump that together with a motor is adapted for establishing a negative pressure when the source of negative pressure 14 is operating, i.e. when it is in an active state or simply "active". The source of negative pressure may comprise any type of pump and motor that are biocompatible or otherwise suitable for use in an NPWT setting and capable of maintaining or drawing adequate and therapeutic vacuum levels. Preferably, the negative pressure level to be achieved is in a range between about -20 mmHg and about -300 mmHg. In some embodiments, a negative pressure range between about -80 mmHg and about - 140 mmHg is used. In some embodiments, a negative pressure range between about -115 mmHg and about -135 mmHg is used. In some embodiments, the negative pressure pump is a diaphragm pump, a peristaltic pump, piezoelectric pump or the like, in which a motor causes the moving parts to draw fluid from the wound site 27.

In the context of the present disclosure, it should be understood that the expressions "negative pressure", "sub-atmospheric pressure", "reduced pressure", or even "vacuum", as used interchangeably herein, generally refer to a pressure less than a local ambient pressure, such as the ambient pressure in a local environment external to a sealed therapeutic environment provided by a wound cover 22. In many cases, the local ambient pressure may also be the atmospheric pressure at which a patient is located. Thus, the "negative pressure" may be understood as a pressure difference between the ambient pressure and the pressure under the wound cover, where ambient pressure is generally set as 0 mmHg. Unless otherwise indicated, values of pressure stated herein are gauge pressures. Similarly, references to increases in negative pressure typically refer to a decrease in (absolute) pressure, while decreases in negative pressure typically refer to an increase in (absolute) pressure.

Further, in some embodiments the apparatus 10 comprises a canister 16 fluidly connected to the negative pressure source 14. The canister 16 may be formed from e.g. moulded plastic or the like, and possibly be a detachable component of the apparatus 10. Moreover, the canister 16 may further be at least partly transparent/translucent to permit viewing into the interior of the canister 16 to assist the user in determining the remaining capacity of the canister 16. As used herein, the term "fluidly connected" should be interpreted broadly and may comprise e.g. any form of tubing, conduits, or channels providing a fluid connection/communication between the canister 16 and the negative pressure source 14 and the dressing 20.

In some embodiments, the canister 16 comprises an inlet port 28 for allowing connection to the tubing 21. The inlet port 28 may also be formed elsewhere at the apparatus 10, however still fluidly connected to the canister 16. The connection between the inlet port 28 and the tubing 21 is a sealed connection, thus ensuring that no leakage is formed at the inlet port 28 during normal operation of the apparatus 10. The tubing 21 is preferably detachably connected to the inlet port 28 through conventional means including a friction fit, bayonet coupling, snap fit, barbed connector, or the like. The inlet port 28 may be moulded/formed from the same material and/or at the same time as forming the canister 16. A similar sealed connection (e.g. using a flange insulation/"O-ring") is formed between the canister 16 (at the outlet port 29) and the source of negative pressure 14. The canister 16 may form a part of the first fluid flow path.

In some embodiments, the apparatus comprises a housing 19 enclosing the negative pressure source 14. The canister 16 may be detachably connected to a housing 19 comprising the negative pressure source 14, whereby e.g. a full canister may be removed and replaced with an empty (new) canister. In such embodiments it may be desirable to provide e.g. the canister 16 and the housing 19 with some form of engagement means for securing the canister 16 to the housing such that the canister 16 is not unintentionally removed from the housing 19. The engagement means may in one embodiment comprise a pair of flexible protrusions extending from the canister 16 and adapted to engage with e.g. corresponding locking grooves provided at the housing 19.

However, in some embodiments, the wound treatment system 1 is a canister-less wound treatment system (not shown), where the wound exudate is collected in an absorbent layer, or the like, of the wound dressing 20. In such embodiments, the fluid flow path from the wound dressing 20 to the negative pressure source 14 is provided with one or more suitable filters (not shown) that are adapted to allow gas flow from the wound site 27 to the source of negative pressure 14 and block the flow of liquids from the wound site 27 to the source of negative pressure 14, as readily understood by the person skilled in the art.

The apparatus 10 further comprises control circuitry 11 (may also be referred to as "a control unit", "a controller", "one or more processors", or the like) operatively connected to the battery 13 and the source of negative pressure 14. The control circuitry 11 is configured to control operation of the source of negative pressure 14. The control circuitry 11 may comprise a microprocessor, microcontroller, programmable digital signal processor or another programmable device. The control circuitry 11 may also, or instead, include an application specific integrated circuit (ASIC), a programmable gate array or programmable array logic, a programmable logic device, or a digital signal processor. Where the control circuitry 11 includes a programmable device such as the microprocessor, microcontroller or programmable digital signal processor mentioned above, the control circuitry 11 may further include computer executable code that controls operation of the programmable device. Thus, in some embodiments, the control circuitry 11 comprises one or more processors and a memory, where the memory contains instructions executable by the processor whereby the apparatus 10 is operative to execute any one of the method steps or functions disclosed herein.

In some embodiments, the apparatus 10 comprises power supply circuitry 13 (or "power supply arrangement") comprising a power source, such as e.g. a battery for powering the apparatus 10. The battery may preferably be of the rechargeable type but may alternatively be arranged to be disposable and thus to be changed once discharged. A specifically adapted battery pack may be used in relation to some embodiments. The apparatus 10 may be of single-use type (allowing use during one single treatment time duration) or multiple-use type (allowing use during several different treatment sessions).

The power supply circuitry 13 may further comprise suitable components to regulate the supply voltage that is provided to various components of the apparatus 10, such as to the source of negative pressure 14, the general control circuitry 11, and so forth. For example, the power supply circuitry 13 may comprise one or more voltage regulators/converters (e.g., boost regulators or buck-boost regulators).

However, in some embodiments, the voltage regulators/converters are provided as a part of the control circuitry 11 depending on requirements. However, in cases where the voltage regulators/converters are provided as a separate component to the control circuitry 11, they are still operatively connected to the control circuitry 11 and therefore controlled by the control circuitry 11.

Furthermore, in some embodiments, the apparatus 10 comprises one or more pressure sensors 15 arranged in fluid connection with the inlet of the source of negative pressure 14, the canister 16, and/or the sealed space 23. In the depicted embodiment of Fig. 1, the apparatus 10 comprises a pressure sensor 15 arranged in a fluid flow path between the canister 16 and the source of negative pressure 14. However, as readily understood by a person skilled in the art, the pressure sensor 15 may be located at any other suitable location to sense or detect a pressure within the fluid flow path between the source of negative pressure 14 and the wound site. For example, the pressure sensor 15 may be arranged within the canister or under the wound cover 22.

During use of the apparatus 10, the wound cover 22 is arranged at a wound site 27 of the user/patient, forming the sealed space 23. A tubing assembly is provided with a first tubing 21 to fluidly connect the wound cover 22 to the inlet port 28 of the apparatus 10. The apparatus 10 is then activated, e.g. by a user pressing a start/pause button 31 of a user-interface of the apparatus 10 that activates the source of negative pressure 14. Alternatively, activation may be initiated via an external device 50 connected to the system 1. The apparatus 10 may for example comprise a suitable communication interface 56 to wirelessly connect to the external device 50.

When activated, the source of negative pressure 14 will start to evacuate air through the canister 16, the inlet port 28, the tubing 21 and the sealed space 23 formed by the wound cover 22. Accordingly, a negative pressure will be created within the sealed space 23. This initial evacuation of air following a start-up of the apparatus 10 may be referred to as "depressurization". The initial evacuation of air (i.e., "depressurization") may continue until reaching a set pressure value (may also be referred to as "target pressure", e.g., -125 mmHg). In some embodiments, the control circuitry 11 is configured to control the source of negative pressure 14 so to establish and maintain a negative pressure level (as measured by the pressure sensor 15) within a negative pressure range. The negative pressure range may for example be -20 mmHg to -300 mmHg, -80 to -180 mmHg, -100 to -150 mmHg, -110 to -140 mmHg, -115 to -135 mmHg, or any suitable subrange thereof (e.g., -80 mmHg to -100 mmHg). The negative pressure range may for example be selected in view of the type of wound and/or the patient.

In case a liquid has been formed at the wound site 27, this liquid from the wound site 27 may at least partly be "drawn" from the wound site, through the tubing 21, the inlet port 28 and into the canister 16. The amount of liquid (possibly defined as exudate) that is drawn from the wound and collected in the canister 16 will depend on the type of wound that is being treated, the duration of the treatment, as well as the type of wound dressing 20 used. For example, in case an absorbent dressing is used, the liquid may be absorbed and collected both in the canister 16 and the wound dressing 20, whereas if a dressing with no absorption capacity or little absorption capacity is used, most or all of the liquid from the wound site 27 may be collected in the canister 16. A suitable filter member (not shown) is generally arranged in proximity to the outlet port 29 of the canister 16 to ensure that no liquid is allowed to pass to the source of negative pressure 14 from the canister 16.

In some embodiments, the wound treatment system 1 further comprise an air inlet port 26 or a controlled leakage port 26 that provides a small and controlled inflow of air (as indicated by the arrows) under the wound cover 22. The air inlet port 26 may for example be provided via a secondary lumen extending from the connector 25 as schematically illustrated. However, in some embodiments the air inlet port 26 may additionally or alternatively be provided at the connector 25 and/or along any suitable location of the tubing 21.

Accordingly, the wound treatment system 1 may in some embodiments be arranged to ensure that a small controlled leakage is present, ensuring that a flow from the sealed space 23 may be achieved (under the assumption that there is no general blocking e.g. within the tubing 21). Additionally, during use of the wound treatment system 1 some leakage may be expected in relation to the wound cover 22. In some embodiments, the air inlet port 26 includes means for supplying/providing air to the wound site 27 at a predetermined supply rate, the rate being from 2 to 7 ml/min, preferably from 3 to 5 ml/min at a predetermined level of negative pressure. The predetermined level of negative pressure may be a value within the range from -80 to -180 mmHg, preferably from -100 to -150 mmHg, more preferably from -110 to -140 mmHg, from -115 to -135 mmHg, or -120 to -135 mmHg. The means for providing air to the wound site 27 (i.e. under the wound cover 22) at a predetermined supply rate may for example be in the form of an air filter (not shown).

In some embodiments, the air filter comprises a hydrophobic and porous material, where the size of the pores is within the range of from 2 to 20 µm, preferably in the range of from 5 to 12 µm. The pore size of the filter is measured in a non-compressed state. In some embodiments, the air filter comprises polyethylene or sintered polyethylene.

As mentioned, the apparatus 10 may comprise a communication interface 56. The communication interface 56 may comprise suitable components (e.g. transceivers, antennas, filters, power amplifiers, etc.) with suitable communication protocols (e.g., Wi-Fi, Bluetooth, Bluetooth Low Energy, Zigbee, or the like) in order to establish a connection with an external device 50 and to transmit and receive wireless signals to and from the external device 50. The communication interface 56 is operatively connected to the control circuitry 11. The communication interface 56 may be configured to transmit and receive wireless signals to and from an external handheld device 50. In more detail, the communication interface 56 comprises one or more transceivers and one or more antennas connected to the one or more transceivers. The one or more transceivers are accordingly configured to transmit signals to an external device 50 via the one or more antennas and to receive signals from an external device 50 via the one or more antennas. For example, the communications interface 56 may include a Wi-Fi transceiver for communicating via a wireless communications network, cellular/mobile phone communications transceiver. In another example, the communication interface 56 may include a short-range wireless transceiver (e.g., a Bluetooth wireless transceiver, etc.) for communicating via a short-range wireless communication network.

The external device may be an external handheld device 50, such as a mobile phone (e.g. a smart phone) operatively connected to the apparatus 10.

Still further, the apparatus 10 may comprise one or more Light Emitting Diodes (LEDs) 62. The one or more LEDs 62 may be considered to form a User Interface (Ul) together with the button 31 arranged on the housing 19. The LEDs 62 may for example be arranged on the housing 19 of the apparatus 10. The LEDs 62 may be arranged under partly transparent portions of the housing to indicate various operating conditions of the apparatus 10. In more detail, the LEDs may be used to indicate a State of Charge (SoC) or available capacity of the power source, presence of a blockage condition, and/or a presence of a leakage condition. The aforementioned transparent portions may accordingly be formed as suitable elements/icons indicating the various operating conditions of the apparatus 10 (e.g., a battery-shaped element/icon for indicating battery capacity). Moreover, each LED may be arranged to emit light of different colours (e.g., green, yellow, red) to provide more granularity in the indications. For example, a green battery icon may indicate high battery capacity (e.g., SoC > 50%), a yellow battery icon may indicate a moderate battery capacity (e.g., 50% > SoC > 10%), while a red battery icon may indicate a low battery capacity (e.g., SoC < 10%). The apparatus 10 may comprise further LEDs than those indicated in the drawings. For example, the apparatus 10 may comprise an LED associated with the button 31 in order to indicate an active state of the apparatus 10.

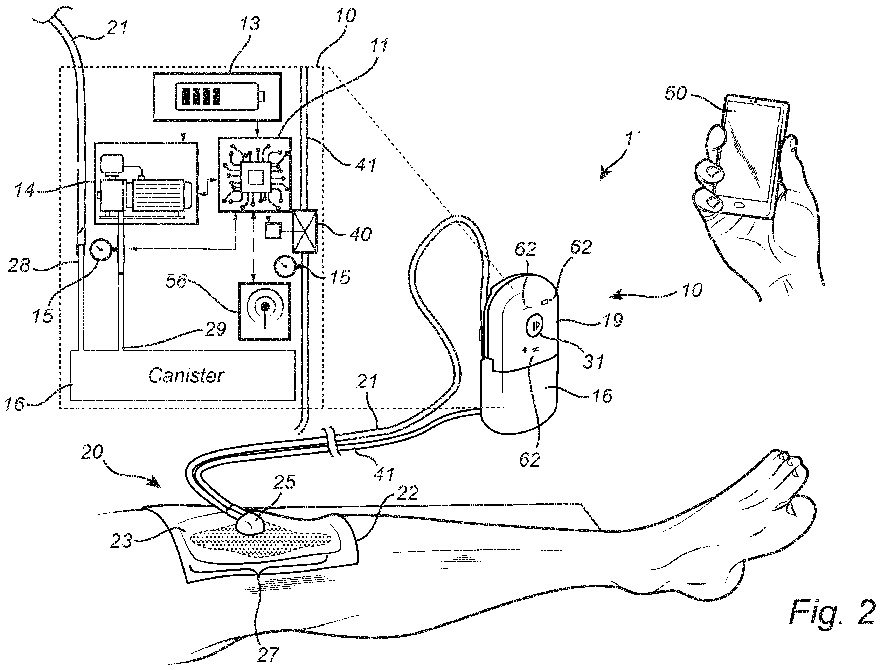

Fig. 2 depicts a wound treatment system 1' in accordance with some embodiments. Many functions, features and components of the wound treatment system 1' depicted in Fig. 2 are the same as or analogous to the wound treatment system 1 depicted in Fig. 1 and will for the sake of brevity and conciseness not be repeated. Instead, the focus will be on the differentiating functions, features, or components between the two wound treatment systems. Thus, as the person skilled in the art readily understands, unless specifically indicated, the foregoing discussion related to the components of the wound treatment system 1 with reference to Fig. 1 is applicable for the corresponding components depicted in Fig. 2 as indicated by the reference numerals.

In general, the wound treatment system 1 depicted in Fig. 1 differs from the wound treatment system 1' depicted in Fig. 2 in that the former is often referred to as a "single-lumen" NPWT system 1 while the latter is often referred to as a "dual-lumen" NPWT system 1'. Accordingly, while the wound treatment system 1 depicted in Fig. 1 has a "controlled inflow of air" via leakage (also referred to as a controlled leakage), the wound treatment system 1' depicted in Fig. 2 does not have a controlled leakage, but instead explicitly controls or regulates the air/fluid supply to the wound site 27 by opening and closing the valve 40.

Therefore, in contrast to the wound treatment system 1 depicted in Fig. 1, the wound treatment system 1' of Fig. 2 comprises a second fluid flow path that fluidly connects the sealed space 23 created by the wound cover 22 to the ambient atmosphere or a "fluid reservoir" (not shown) via an electronically controllable valve 40. The second fluid flow path (may also be referred to as "air lumen") is defined by a second tubing 41 that may be of any suitable flexible tubing fabricated from elastomeric or polymeric materials. Here, the fluid flow path from the inlet of the source of negative pressure 14 to the wound dressing 20 may be construed as a "first" fluid flow path or "exudate lumen".

Here, the control circuitry 11 is electronically connected to the electronically controllable valve 40, and the control circuitry 11 is further configured to control an operation of the electronically controllable vale 40 so to release negative pressure from the wound site via the second fluid flow path (or "air lumen") 41. In other words, the control circuitry 11 is configured to open the electronically controllable valve 40 so to introduce fluid (e.g. air) to the wound site 27 when there is a sub-atmospheric pressure under the wound cover 22. This operation may also be referred to as "flushing".

Since the wound treatment system 1' depicted in Fig. 2 does not have any "controlled leakage flow", there is a risk that the apparatus 10 would not be able to draw any, or at least a sub-optimal amount of "exudate" from the wound site 27 to the canister 16 once negative pressure has been established under the wound cover 22 due to lack of airflow through the system 1'. Therefore, in order to be able to draw desired amounts of wound exudate from the wound site, a fluid, such as e.g. air from the ambient atmosphere, may be controllably introduced at defined time intervals and/or in response to pressure measurements by opening the electronically controllable valve 40. Once enough fluid has been introduced (e.g. in response to a negative pressure within the first and/or second fluid flow path reaching a lower threshold), the control circuitry 11 is configured to close the electronically controllable valve 40 and to activate the source of negative pressure 14.

The ability to controllably "flush" the system 1' by injecting air via an electronically controllable valve 40 may provide the advantage of longer pressure regulation cycles, which reduces the on-time for the source of negative pressure 14 and therefore reduces the energy consumption of the apparatus 10.

As before, the apparatus 10 of the wound treatment system 1' may comprise one or more pressure sensors 15 arranged in fluid connection with the inlet of the source of negative pressure 14, the canister 16, and/or the sealed space 23. However, the apparatus 10 may alternatively or additionally comprise a pressure sensor 15 arranged in fluid connection with the second fluid flow path (i.e., "air lumen"). The pressure sensor 15 (i.e., "air lumen pressure sensor") is preferably arranged to measure a pressure level in the second fluid flow path downstream of the electronically controllable value 40 (i.e., between the valve 40 and the wound site 27). In the depicted embodiment of Fig. 2, the apparatus comprises two pressure sensors 15, a first pressure sensor 15 configured to measure a pressure level within the first fluid flow path, and a second pressure sensor 15 configured to measure a pressure level within the second fluid flow path. However, the apparatus 10 may comprise a single pressure sensor arranged in any one of the suitable locations mentioned in the foregoing.

The apparatus 10 may further comprise an air filter (not shown) arranged in the second fluid flow path in order to limit the airflow when the electronically controllable valve 40 is open. In some embodiments, the air filter comprises a hydrophobic and porous material, where the size of the pores is configured to allow for a flow in the range of 50-120 ml/s, such as 60-100 ml/s when the valve 40 is opened after negative pressure has been established under the wound cover 20. In other words, the size of the pores is configured to allow for a flow in the range of 50-120 ml/s, such as 60-100 ml/s when the valve 40 is opened and when there is a specific negative pressure level within the system 1'. The pore size of the filter is preferably measured in a non-compressed state. In some embodiments, the air filter comprises polyethylene or sintered polyethylene. The air filter may also serve to reduce the risk of contaminants entering the wound site 27 when the valve 40 is opened (i.e., during "flushing"). The air filter may be arranged in the tube 41 that at least partly defines the second fluid flow path. The air filter may be arranged upstream of the valve 40 (i.e., between the valve 40 and the inlet of ambient air, or downstream of the valve 40 such as between the valve and the air lumen pressure sensor 15.

Moving on, regardless of the wound treatment system 1, 1' being single lumen (Fig. 1) or dual lumen (Fig. 2), the control circuitry 11 is configured to activate the source of negative pressure 14 in order to establish a set target pressure at the wound site 27, and to store a plurality of pressure values, as measured by the pressure sensor 15, while the source of negative pressure is active.

Further, the control circuitry 11 is configured to configure one or more control parameters of the apparatus based at least in part on the stored plurality of pressure values. The one or more control parameters may for example comprise a supply voltage of the source of negative pressure 14, the target pressure, a pressure range for pressure regulation, a pressure cycle duration, a therapy duration, a flush duration (or "flushing period" duration), and/or a pressure range for the flushing sequence.

A "pressure cycle" or "pressure regulation cycle" may be understood as a time period comprising at least a "pressure regulation period" and following "flushing period" of a dual lumen NPWT system 1'. A "pressure regulation period" (may be referred to as "inter-flushing period") that defines a time between two consecutive flushing periods (and eventually the time between the end of the initial depressurization from atmospheric to therapeutic pressure and the first flushing period). Thus, the duration of a pressure cycle ("pressure cycle duration") may for example be 10 minutes, 20 minutes, 30 minutes, 60 minutes or the like depending on for example the wound characteristics and/or the dressing type. The therapy duration defines a time period from the initial activation of the apparatus 10 to the end of the prescribed therapy.

This process of activating the source of negative pressure 14, storing pressure values, and configuring the one or more control parameters of the apparatus 10 may advantageously be executed following a start-up of the apparatus 10 when therapy is to be initiated. Thus, the "activation" may be the initial activation of the source of negative pressure 14 ("depressurization"). An advantage of executing the process to configure the control parameter at this stage is that the apparatus 10 is allowed to configure itself already from the start when the apparatus 10 is put to use. Accordingly, the control circuitry 11 may be configured to activate the source of negative pressure in order to establish a set target pressure at the wound site from an atmospheric pressure level.

However, this activation may be an activation of the source of negative pressure 14 during pressure maintenance or regulation at the wound site 27. In other words, the apparatus 10 may change its settings by configuring the one or more control parameters of the apparatus 10 at any time during therapy. This may be advantageous to allow the apparatus 10 to potentially correct any setting should it have had a non-optimal configuration at an initial stage of the therapy.

Moreover, the control circuitry 11 may be configured to store the plurality of pressure values, as measured by the pressure sensor 15, for a set duration of time while the source of negative pressure 14 is active. The set duration of time may be a duration of time suitable to derive a pressure curve profile and may accordingly depend on the type of source of negative pressure used. The set duration of time may be at least 2 seconds, at least 5 seconds, or at least 10 seconds. The duration of time may be the time required for the apparatus 10 to complete the initial depressurization. In other words, the duration of time may be the time it takes the source of negative pressure 14 to establish a target pressure level (e.g., -125 mmHg) at the wound site 27. Alternatively, the duration of time may be the time it takes the source of negative pressure to reach a threshold pressure level as measured by the pressure sensor 15. The threshold pressure level may be different from the target pressure level. In particular, the threshold pressure level may be higher than the target pressure level, such as for example -30 mmHg, -40 mmHg, -50 mmHg or -60 mmHg.

Accordingly, depending on the pressure characteristics of the system 1, 1', as reported by the pressure sensor over time, the apparatus 10 is configured to configure its setting to provide a customized configuration for the current situation or scenario that it is being used. Thereby, the probability of having a more optimized therapy being applied in a variety of potential use cases may be improved. In more detail, the pressure level behaviour over time may be used as an indication that the apparatus 10 is connected to a particular type of wound dressing 20 or that it is connected to multiple wound dressings 20. Different types of wound dressings 20 may be of different sizes and shapes that affect how the pressure decreases in the system 1, 1'. Thus, for larger wound dressings, the apparatus 10 may need to increase the speed of the source of negative pressure 14 or change the thresholds for controlling the pressure regulation in order to better optimize the negative pressure wound therapy.

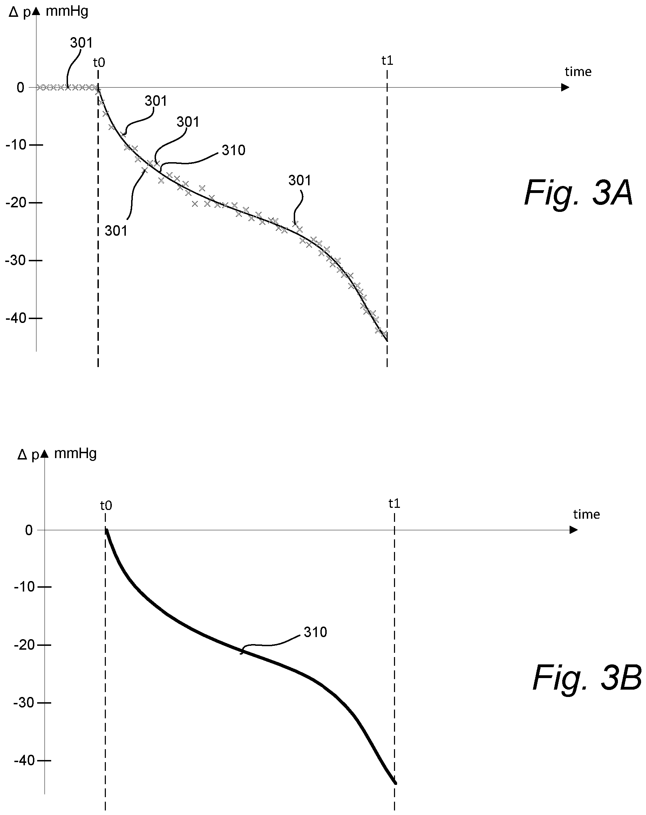

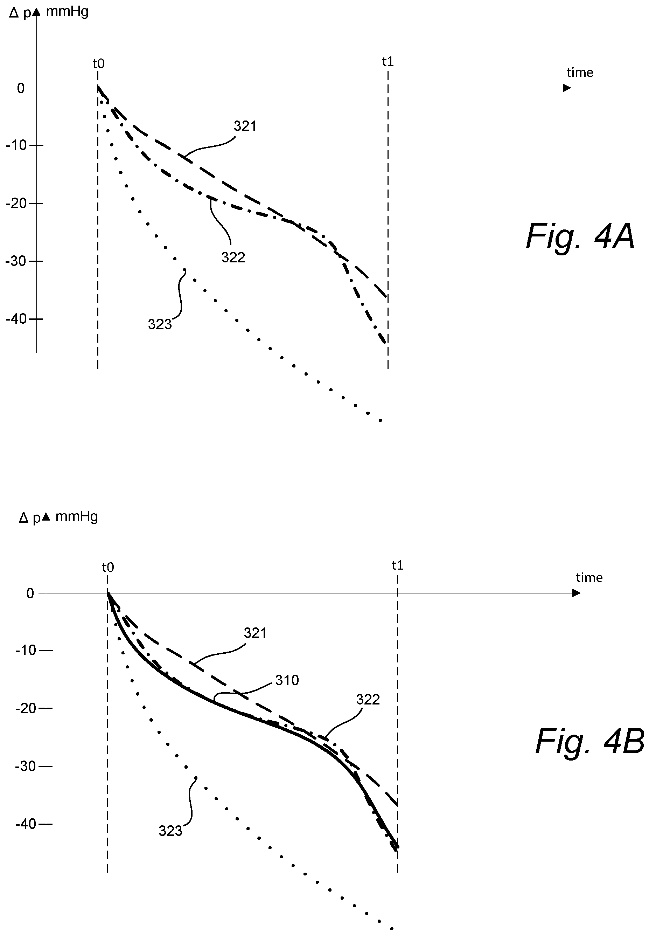

Turning now to Figs. 3A, 3B, 4A, and 4B which are graphs of negative pressure over time showing schematic illustrations of example pressure curves in accordance with some embodiments. In some embodiments, the stored plurality of pressure values 301 at least in part defines a pressure curve profile 310. Moreover, the control circuitry 11 may be configured to select configuration profile out of a plurality stored or otherwise predefined configuration profiles based on the stored plurality of pressure values 301. Each configuration profile of the plurality of configuration profiles may be associated with a corresponding pressure curve profile. Each configuration profile defines settings for the one or more control parameters of the apparatus 10. Thus, the selection of a configuration profile may accordingly be based on a comparison between the pressure curve profile 310 defined at least in part by the stored plurality of pressure values 301 and the pressure curve profiles of the plurality of configuration profiles.

Further, the control circuitry 11 may be configured to determine a type of wound dressing 20 out of a plurality of predefined types of wound dressings based on the pressure curve profile 310. The control circuitry 11 may therefore be configured to configure the one or more control parameters of the apparatus 10 based at least in part on the determined type of wound dressing 20 or selected configuration profile.

Thus, each type of wound dressing out of the plurality of predefined types of wound dressings, or each configuration profile out of the plurality of predefined configuration profiles, may be associated with a corresponding pressure curve profile 321, 322, 323. Thus, the control circuitry 11 may be configured to determine the type of wound dressing or the applicable configuration profile by comparing the pressure curve profile 310 defined at least in part by the stored plurality of pressure values 301 to the corresponding pressure curve profiles 321, 322, 323 of the plurality of predefined types of wound dressings or configuration profiles, selecting the corresponding pressure curve profile 321, 322, 333 that is a closest match to the pressure curve profile 310 defined at least in part by the stored plurality of pressure values 301, and selecting the type of wound dressing associated with the selected corresponding pressure curve profile 321, 322, 323 as the determined type of wound dressing.

As mentioned, different types of wound dressings 20 may be of different sizes and shapes that affect how the pressure decreases in the system 1, 1', and therefore affect the pressure curve profile exhibited by the system 1. 1' when the source of negative pressure 14 is activated. However, other aspects or features of the wound dressing 10 may affect the pressure curve profile. For example, the transmission rate moisture vapor transmission rate (MVTR) of a backing layer 22 of the wound dressing 20 may affect the pressure curve profile. The backing layer may for example have a moisture vapor transmission rate (MVTR) in the range of from 500 to 3500 g/m²/24h, as measured by NWSP070.4R0(15). Moreover, if the wound dressing 20 comprises an absorbent layer or structure with superabsorbent polymer (SAP) particles, the amount of SAP particles may affect the pressure curve profile. The absorbent layer or structure may for example comprise SAP particles in the amount of 10 to 20 mg/cm². Accordingly, the pressure curve profile exhibited by the system 1. 1' when the source of negative pressure 14 is activated may depend on a size of the wound dressing, a shape of the wound dressing, an MVTR of a backing layer of the wound dressing and/or an amount of SAP particles in the wound dressing.

Moreover, the presence of a wound filler (such as a gauze or foam such as polyurethane foam) and the type of wound filler may affect how the pressure decreases in the system 1, 1', and therefore affect the pressure curve profile exhibited by the system 1. 1' when the source of negative pressure 14 is activated.

Further, the part of the body where the wound is located may also affect the pressure curve profile as more or less parasitic leakage may be present depending on the placement of the wound dressing on the patient's body. Moreover, different types of wound may exhibit different pressure curve profiles due to their variety in shape. Thus, the selected configuration profile for configuring of the control parameters of the apparatus 10 need not necessarily only be related to the type of wound dressing that is applied, but it may additionally or alternatively be related to the type of wound and/or the position of the wound on the body of the patient.

In some embodiments, each pressure curve profile may comprise one or more metrics or parameters defining a curve shape, a time to target pressure, a pressure rate of change, or one or more inflection points. The one or more inflection points may be defined by a second derivative of the pressure curve.

Accordingly, Fig. 3A is a schematic illustration of a graph of negative pressure over time showing a plot of a plurality of pressure values 301, as measured by the pressure sensor 15, for a duration of the time that the source of negative pressure 14 is active. The sampling rate of the sensor 15 is assumed to be consistent and known so that the measurements can be related to time in a proper manner. Furthermore, the data (pressure measurements) may be pre-processed in order to for example identify and handle missing values (e.g., using interpolation if the data is sparse), to filter noise (e.g., by applying a low-pass filter to remove high-frequency noise or fluctuations), and/or to normalize or scale the data.

The data (pressure measurements 301) are plotted against time, providing an initial visualization of a pressure curve. Next, in order to derive the pressure curve 310, one may fit the data to a known or otherwise predefined mathematical model, such as a polynomial or exponential equation. Fig. 3A depicts the resulting pressure curve profile 310 derived from the plurality of pressure measurements 301, and Fig. 3B depicts the resulting pressure curve profile 310 without the pressure values 301.

Further, Fig. 4A is a schematic illustration of three pressure curve profiles 321, 322, 323, each of them being associated with a corresponding type of wound dressing. In other words, the three pressure curve profiles 321, 322, 323 are the resulting curve profiles of a set of pressure measurements made using three different types of wound dressings. These pressure curve profiles 321, 322, 323 may for example be stored in a memory of the apparatus 10 and generated from empirical data.

As mentioned, the control circuitry 11 may be configured to compare the pressure curve profile 310 defined at least in part by the stored plurality of pressure values 301 to the corresponding pressure curve profiles 321, 322, 333 of the plurality of predefined types of wound dressings. This process is schematically indicated in Fig. 4B showing the four different pressure curve profiles 310, 321, 322, 323 in the same frame of reference.

In more detail, the comparison of the pressure curve profile 310 to the predefined pressure curve profiles 321, 322, 323 may comprise measuring a similarity or distance between the pressure curves. For example, one may measure a Euclidean distance between the pressure curve profile 310 and each of the predefined pressure curve profiles 321, 322, 323, by measuring the overall point-wise different between the curves. Alternatively, one can calculate the Mean Squared Error (MSE) by calculating an average squared difference between corresponding points of the curves. If, the different pressure curves 310, 321, 322, 323 are represented as vectors of pressure values sampled at uniform time intervals, one can determine a cosine similarity by measuring the angle between the vector representations of the curves. Moreover, the comparison may include comparing defining a curve shape, a time to target pressure, a pressure rate of change, or one or more inflection points of the pressure curve profiles.

Then, to select the corresponding pressure curve profile 321, 322, 333 that is a closest match to the pressure curve profile 310 defined at least in part by the stored plurality of pressure values, the control circuitry 11 may be configured to select the corresponding pressure curve profile 321, 322, 333 that has the highest similarity (e.g., if cosine similarity is used) or the lowest distance (if Euclidian distance or MSE is used). In the depicted embodiment, the pressure curve profile 322 indicated by the dashed and dotted line is the closest match to the pressure curve profile 310 defined at least in part by the stored plurality of pressure values.

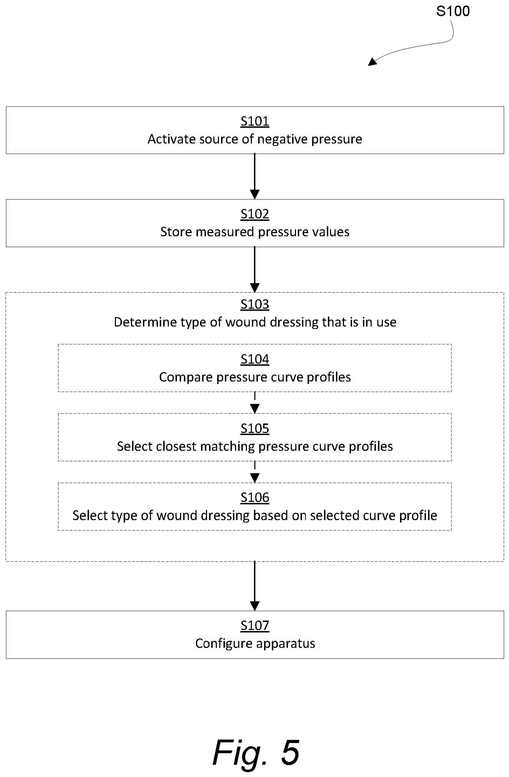

Fig. 5 is a schematic flowchart representation of a method S100 for controlling an apparatus 10 for providing negative pressure to a wound site 27. The apparatus 10 may be an apparatus according to any one of the embodiments disclosed herein. The method S100 is preferably a computer-implemented method S100, performed by a processing system of the apparatus 10. The processing system may for example comprise one or more processors 11 and one or more memories coupled to the one or more processors 11, wherein the one or more memories store one or more programs that perform the steps, services and functions of the method S100 disclosed herein when executed by the one or more processors.

The method S100 comprises activating S101 the source of negative pressure in order to establish a set target pressure at the wound site, and storing S102 a plurality of pressure values, as measured by the pressure sensor, while the source of negative pressure is active. Further, the method S100 comprises configuring S107 one or more control parameters of the apparatus based at least in part on the stored S102 plurality of pressure values.

Moreover, in some embodiments, the stored S102 plurality of pressure values at least in part defines a pressure curve profile. The method S100 may further comprise determining S103 a type of wound dressing out of a plurality of predefined types of wound dressings based on the pressure curve profile. Accordingly, the configuration S107 of the one or more control parameters of the apparatus may be based on the determined S103 type of wound dressing.

Further, in some embodiments, the determination S103 of the type of wound dressing may comprise comparing S104 the pressure curve profile defined at least in part by the stored plurality of pressure values to the corresponding pressure curve profiles of the plurality of predefined types of wound dressings, selecting S105 the corresponding pressure curve profile that is a closest match to the pressure curve profile defined at least in part by the stored plurality of pressure values, and selecting S106 the type of wound dressing associated with the selected corresponding pressure curve profile as the determined type of wound dressing.

The determination S103 of the type of wound dressing may be understood as selecting a configuration profile out of a plurality stored or otherwise predefined configuration profiles based on the pressure curve profile defined at least in part by the stored S103 plurality of pressure values. Each configuration profile comprises settings for the one or more control parameters of the apparatus.

In more detail, the method S100 may comprise the pressure curve profile defined at least in part by the stored plurality of pressure values to the corresponding pressure curve profiles of the plurality of predefined configuration profiles, selecting S105 the corresponding pressure curve profile that is a closest match to the pressure curve profile defined at least in part by the stored plurality of pressure values, and selecting S106 the configuration profile associated with the selected corresponding pressure curve profile. The configuration S107 of the one or more control parameters of the apparatus may be based on the selected configuration profile.

Executable instructions for performing these functions are, optionally, included in a non-transitory computer-readable storage medium or other computer program product configured for execution by one or more processors of an apparatus for providing reduced pressure to a wound dressing.

The herein disclosed technology has been presented above with reference to specific embodiments. However, other embodiments than the above described are possible and within the scope of the claims. Different method steps than those described above, performing the method by hardware or software, may be provided within the scope of the claims. Thus, according to some embodiments embodiment, there is provided a non-transitory computer-readable storage medium storing one or more programs configured to be executed by one or more processors of an apparatus of a reduced-pressure wound treatment system, the one or more programs comprising instructions for performing the method according to any one of the above-discussed embodiments.

Generally speaking, a computer-accessible medium may include any tangible or non-transitory storage media or memory media such as electronic, magnetic, or optical media coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) 11 (associated with the apparatus 10) may be or include any number of hardware components for conducting data or signal processing or for executing computer code stored in memory. The device 10 may accordingly have an associated memory, and the memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to some embodiments, any distributed or local memory device may be utilized with the systems and methods of this description. According to some embodiments embodiment the memory is communicably connected to the processor 11 (e.g., via a circuit or any other wired, wireless, or network connection) and includes computer code for executing one or more processes described herein.

It should be noted that any reference signs do not limit the scope of the claims, that some embodiments may be at least in part implemented by means of both hardware and software, and that several "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the appended claims. Likewise, software implementations could be accomplished with standard programming techniques with rule-based logic and other logic to accomplish the generating steps, activating steps, operating steps, causing steps, steps. The above mentioned and described embodiments are only given as examples and should not be limiting to the appended claims. Other solutions, uses, objectives, and functions within the scope of the below described patent claims should be apparent for the person skilled in the art.

## Claims

1. An apparatus (10) for providing reduced pressure to a wound site (27) covered by a wound dressing (20), the apparatus comprising:
a source of negative pressure (14) configured to provide negative pressure via a first fluid flow path from an inlet of the source of negative pressure (14) to the wound site (27);
a pressure sensor (15) configured to monitor a pressure level in a fluid flow path that is in fluid connection with the wound site (27) or to monitor a pressure level at the wound site (27);
control circuitry (11) operatively connected to the source of negative pressure (14) and to the pressure sensor (15), wherein the control circuitry (11) is configured to:
activate the source of negative pressure (14) in order to establish a set target pressure at the wound site (27);
store a plurality of pressure values (301), as measured by the pressure sensor (15), while the source of negative pressure (14) is active; and
configure one or more control parameters of the apparatus (10) based at least in part on the stored plurality of pressure values.

2. The apparatus (10) according to claim 1, wherein the stored plurality of pressure values (301) at least in part defines a pressure curve profile (310), and wherein the control circuitry (11) is configured to:
determine a type of wound dressing out of a plurality of predefined types of wound dressings based on the pressure curve profile (310);
configure the one or more control parameters of the apparatus (10) based at least in part on the determined type of wound dressing.

3. The apparatus (10) according to claim 2, wherein each type of wound dressing out of the plurality of predefined types of wound dressings is associated with a corresponding pressure curve profile (321, 322, 333).

4. The apparatus (10) according to claim 3, wherein the control circuitry (11) is configured to determine the type of wound dressing by:
comparing the pressure curve profile (310) defined at least in part by the stored plurality of pressure values (301) to the corresponding pressure curve profiles (321, 322, 333) of the plurality of predefined types of wound dressings;
selecting the corresponding pressure curve profile (321, 322, 333) that is a closest match to the pressure curve profile (310) defined at least in part by the stored plurality of pressure values;
selecting the type of wound dressing associated with the selected corresponding pressure curve profile (321, 322, 323) as the determined type of wound dressing.

5. The apparatus (10) according to any one of claims 2-4, wherein each pressure curve profile (310, 321, 322, 323) comprises one or more parameters defining a curve shape, a time to target pressure, a pressure rate of change, or one or more inflection points.

6. The apparatus (10) according to claim 5, wherein the curve shape is defined by an exponential equation or a polynomial equation.

7. The apparatus (10) according to claim 5 or 6, wherein the one or more inflection points are defined by a second derivative of the pressure curve.

8. The apparatus (10) according to any one of claims 1-7, further comprising:
an electronically controllable valve (40) configured to release negative pressure from the wound site (27) via a second fluid flow path;
wherein the pressure sensor (15) is arranged to monitor a pressure level in the second fluid flow path.

9. The apparatus (10) according to any one of claims 1-7, wherein the pressure sensor (15) is arranged to monitor a pressure level in the first fluid flow path.

10. The apparatus (10) according to any one of claims 1-9, wherein the one or more control parameters comprises a supply voltage of the source of negative pressure (14), the target pressure, a pressure range for pressure regulation, a pressure cycle duration, or a therapy duration.

11. A computer-implemented method (S100) for controlling an apparatus comprising a source of negative pressure configured to provide negative pressure via a first fluid flow path from an inlet of the source of negative pressure to the wound site covered by a wound dressing and a pressure sensor configured to monitor a pressure level in a fluid flow path that is in fluid connection with the wound site or to monitor a pressure level at the wound site, the computer-implemented method comprising:
activating (S101) the source of negative pressure in order to establish a set target pressure at the wound site;
storing (S102) a plurality of pressure values, as measured by the pressure sensor, while the source of negative pressure is active; and
configuring (S107) one or more control parameters of the apparatus based at least in part on the stored plurality of pressure values.

12. The computer-implemented method (S100) according to claim 11, wherein the stored (S102) plurality of pressure values at least in part defines a pressure curve profile, and wherein the method comprises:
determining (S103) a type of wound dressing out of a plurality of predefined types of wound dressings based on the pressure curve profile;
configuring (S107) the one or more control parameters of the apparatus based on the determined type of wound dressing.

13. The computer-implemented method (S100) according to claim 12, wherein each type of wound dressing out of the plurality of predefined types of wound dressings is associated with a corresponding pressure curve profile.

14. A computer program product comprising instructions which, when the program is executed by a computing device of an apparatus for providing negative pressure to a wound site, causes the apparatus to carry out the method (S100) according to any one of claims 11-13.

15. A system (1) comprising:
an apparatus (10) according to any one of claims 1-10;
the wound dressing (20); and
a tubing assembly defining the first fluid flow path.
